# EUROPEAN PATENT APPLICATION

(11) **EP 2 404 633 A1**
(43) Date of publication of application: **11.01.2012**
(21) Application number: 10168669.9
(22) Date of filing: 07.07.2010
(51) Int. Cl.: A61M 5/315

(54) **Drug delivery device and system of drug delivery devices**

(71) Applicant: Sanofi-Aventis Deutschland GmbH, 65929 Frankfurt am Main (DE)
(72) Inventor: Smith, Christopher James, Cheshire, CW4 7QG (GB); Mackellar, Alasdair, WA15 9PH Cheshire (GB)

(57) **Abstract**

A system of injection devices (1) is provided where each device has preset minimum and maximum possible dose settings to control delivery of a medicament to a patient. A set dose less than the predetermined minimum will not be deliverable and hard stops will prevent setting of a dose greater than a predetermined maximum.

## Description

The present invention is generally directed to dose setting mechanisms for drug delivery devices that control minimum and/or maximum possible dose settings. More particularly, the present invention is generally directed to drug delivery devices, such as pen type drug delivery devices where therapy demands that a patient receive at least a certain minimum dose and not exceed a certain maximum dose of a particular medicament. Such devices provide for self-administration of medicinal product from a multi-dose cartridge and contain dose limiting mechanisms setting. The present invention may find application in both disposable and reusable type drug delivery devices. However, aspects of the invention may be equally applicable in other scenarios as well.

Self administered injectable medicaments are often delivered using a variable-dose injection device. Prior to the injection the user selects the dose that they require according to their prescribed dose and/or their current or expected future physical condition. A typical example would be an insulin delivery device for diabetics where a patient's dose is determined according to their prescribed dose and their expected food intake and activity level. Typically such devices allow the user to select any dose from 1 unit up to the maximum units that the device can deliver, typically 60 units or 80 units for a manual device, such as a pen-type or syringe injection device.

Pen type drug delivery devices have been design and developed to perform regular injections by persons without formal medical training. This is increasingly common among patients having diabetes where self-treatment enables such patients to conduct effective management of their disease. Because the patient, and not the health care worker, is using such a drug delivery device, one requirement is that the device must be robust in construction. The drug delivery device must also be easy to use both in terms of the drug delivery device manipulation and understanding of the device's operation.

This is especially true for diabetics who are required to inject themselves repeatedly with insulin solution and the volume of insulin to be injected may vary from patient to patient and even from injection to injection. For at least this reason, certain diabetics may require drug delivery devices that allow the patient to inject successive measured dosages of the same or perhaps different preset volumes of insulin solution accurately and with minimum dexterity challenges. This presents a further design challenge since, in the case of certain diabetics, users may have impaired vision and/or may be physically infirm with limited dexterity.

In addition to insulin, other medicaments require a minimum dose to be delivered before they are therapeutically effective. A variable-dose device that allows the patient to deliver doses below the therapeutically effective minimum dose creates the possibility that the user may deliver the ineffective doses either by an error of dose calculation or by mistakenly selecting the incorrect dose. Likewise, some medicaments require that a maximum dose is not to be exceeded. This may be for safety reasons such as increased risk or severity of side-effects or excessive or unwanted actions of the medicament. Current variable-dose delivery device typically have a maximum dose that is limited by the maximum dose that the delivery mechanism can provide, however, this does not necessarily relate to the maximum advised or prescribed dose of the medicament.

There is, therefore, a general need to reduce or eliminate the risk that a user of an injection device will set a dose either below the minimum effective dose or above a safe maximum dose of a particular medicament.

This problem is essentially solved by a drug delivery device with the features of claim 1. Preferred embodiments are defined in the sub-claims.

According to the invention the drug delivery device comprises a medicament reservoir attachable to a dose setting module and/or housing. The dose setting module is configured to only allow delivery of a set dose if the set dose is equal to or greater than a predetermined minimum dose X of the medicament. Further, the dose setting module is configured to prevent a user from setting a dose that exceeds a predetermined maximum dose Y of the medicament, where X is greater than zero. In other words, a set dose less than the predetermined minimum will not be deliverable and hard stops will prevent setting of a dose greater than a predetermined maximum. Preferably, from dose X to dose Y equals a therapeutic range of medicament prescribed by a physician. In addition, a system or "family" of injection devices may be provided where each device has preset minimum and maximum possible dose settings to control delivery of a medicament to a patient.

The present invention has at least two applications. First, is the delivery of a single active medicament which must be a variable dose within a defined dose window, i.e. the dose must be more than a certain minimum dose and must not exceed a certain maximum dose. The second application relates to the delivery of a combined formulation of active medicaments where at least one of the medicaments is preferably delivered as a variable dose and at least one other medicament is preferably delivered as a fixed dose, and where this fixed dose can safely be allowed to vary within a defined dose window, for example by ±10% of the nominal fixed dose.

The minimum and/or maximum dose limited delivery device of the present invention could be used for any medicament that requires a minimum dose to be delivered before it becomes therapeutically effective, but where a degree of dose adjustment may be required. This dose adjustment may be required for a number of reasons, including tailoring a dose to a patient's body weight or the severity of their medical condition. The minimum and maximum dose limited device (min/max device) may also be used instead of a fully variable (i.e., 0 to max dose) device in order to reduce the possibility for dosing errors by the patient. Using the min/max device rather than a variable dose pen reduces the risk that a patient might accidentally deliver a dose outside the defined dose window, i.e., either too high or too low.

One example of the utility of the present invention is where a parent could give the min/max delivery device to a child for the child to self-administer and the parent would know that the minimum and maximum levels of the min/max device limited the possible severity of any overdose or under dose. Another example of where such a device might be applicable is for patients who take long acting insulin. Typically a variable dose pen is required when a patient is "titrating" their dose to reach their target blood glucose level. However, once the target blood glucose level has been achieved the dose of long acting insulin typically remains more or less constant over relatively long periods of time. During this period, where their insulin dose is either constant or changes by only a few units on a day-to-day basis, the patient's long acting insulin needs could be effectively met by the minimum and maximum dose limited delivery device.

Table 1 (shown below) shows an example family or system of delivery devices, "Pen 1" through "Pen 4", which could be used in place of a single 1-80 unit variable dose device. Each of the Pens 1 - 4 are designed and manufactured around the same basic mechanism, but each pen contains either additional or alternative components which are used to set a different minimum and maximum dose. Patients would be prescribed a particular Pen according to their stable long acting insulin dose. For example, according to Table 1 a patient prescribed 30 units per day of long acting insulin would be prescribed Pen 2, which has a minimum dose of 22 units and a maximum dose of 40 units, respectively. Any number of mechanical components can be used in such a pen design to ensure these predetermined min/max doses, including axial and/or stops, detents, clutches, compressible fingers, or the like components.

The insulin dose of diabetic patients may change gradually over time. Therefore, there may be a small amount of dose range overlap between Pens to allow for a smooth transition between pens as the dose increases. For example, according to Table 1 a patient prescribed 40 units per day of long acting insulin would be given Pen 2 if they expected their dose to decrease over time or Pen 3 if they expected their dose to increase over time. The number of pens in the system of pens (pen "family") and the selected dose ranges shown in Table 1 are illustrative only. By using the min/max devices of our invention a mistake when selecting the dose is limited to within the pen's operating window. Dialing a dose above or delivering a dose below the pen's dose range would not be possible and this would alert the patient to their error.

The min/max device may also be applicable for the delivery of other medicines, particularly where there is a risk of confusion with similar devices that may lead to dose errors or drug / device mix-ups. One such example would be rapid acting insulin and long acting insulin. Both of types of insulin are measured in "units" however the same number of units of each insulin type will have a very different effect and a patient will be described different doses of each drug to be taken at different times throughout the day.

A mix up of long acting and rapid acting insulin can cause hypoglycemia and is potentially fatal. Both types of insulin may be delivered using injection pen devices of the type mentioned above. Patients perform their injections on such a routine basis that an "automatic pilot" effect can occur where patients have been known to mix up their insulin pens, even though the pens are of different design, color, shape and carry different labels.

The min/max device according to the invention may help to prevent this mix up from occurring. For example, assume both rapid acting and long acting insulin were each provided with a family of min/max devices according to Table 1. A patient is prescribed 50 units per day of long acting insulin (which would require long acting Pen 3) and 15 units of rapid acting insulin with meals (which would require Pen 1). The most dangerous mix up would occur if the patient mistakenly delivered 50 units of rapid acting insulin rather than long acting insulin. If the patient attempted to do this with the min/max devices then the patient would pick up the rapid insulin device (Pen 1) and find that they could not dial beyond 24 units. This should alert them to the fact that this is not the correct insulin pen, and therefore the incorrect insulin type, and prevent the incorrect insulin being delivered.

Tables 1 - 3 illustrate some of the following possible systems of injection device combinations. For example, Table 2 shows a system of two or more drug delivery devices wherein a first drug delivery device and a second drug delivery device each have a different therapeutic range of dose X to dose Y, where X is the minimum allowed dose, which is greater than zero, and Y is the maximum allowed dose, which is less that the maximum dose that each injection device is designed to deliver. Specifically, Table 2 (shown below) shows the situation where a system of injection devices where the therapeutic range of each drug delivery device do not overlap the therapeutic range of the other drug delivery devices in the system. The therapeutic range of the drug delivery devices can be a subset of the therapeutic ranges of other devices in the system. Table 3 (shown below) illustrates a particular system where the minimum dose X for all devices in the system is the same, i.e., 10 units. Tables 4-6 (shown below) represent the same systems as shown in Tables 1-3 except those devices have the mechanical capability to dial a priming dose.

The min/max concepts may be applied equally to both disposable devices and reusable devices.

Certain medicines also require the user to perform a "priming" dose to confirm the correct operation of the delivery device and needle. This is usually accomplished by delivering an "air-shot" of 2 units and then checking that the medicine can be seen coming out of the needle. The min/max concept shown in Table 1 would not permit this. If priming functionality is required a second permissible "dose window", for example ranging from 1-2 units, may also be implemented within each pen mechanism. An example of how this could be applied is shown in Tables 4-6. Although the Tables show only even numbers of units this is done only for clarity and the device may be configured to deliver odd and even units or potential ½ units.

As mentioned, the present invention is also useful in therapies where the delivery of a combined formulation of active medicaments is needed, where at least one of the medicaments is preferably delivered as a variable dose and at least one other medicament is preferably delivered as a fixed dose. If a patient requires a combination of medicines then there is an advantage if those medicines can be provided as a single formulation (i.e. both drugs are mixed together in predefined proportions and supplied in one primary pack or cartridge) for delivery by a single injection device in one injection through a single needle. However, if one of the drugs requires the delivery of a user-selectable variable dose and the second drug requires a dose above a minimum dose to be therapeutically effective and must not exceed a given maximum dose, then it is beneficial for the drug delivery device to be configured such that it is prevented from delivering doses that are outside of this range.

For example, if a patient is prescribed a combination therapy of long acting insulin (typically delivered in variable dose devices) and GLP-1 (typically delivered as a fixed dose). GLP-1 is a glucagon-like peptide-1, which is derived from the transcription product of the proglucagon gene and is found in the body where it is secreted by the intestinal L cell as a gut hormone. GLP-1 possesses several physiological properties that make it (and its analogs) a subject of intensive investigation as a potential treatment of diabetes mellitus. In order to avoid the patient having to perform two injections, one of insulin and one of GLP-1, the two medicines are pre-mixed into a single formulation. Since both medicines are pre-mixed in a fixed ratio it is not possible to vary the long acting insulin dose without also varying the GLP-1 dose. However, it may be acceptable for the GLP-1 dose to vary within a given tolerance, for example ±10%, around a fixed nominal dose. It is therefore possible, using a family of min/max limited devices to provide a family of pre-mix devices, where between them will allow delivery of a variable long acting insulin dose and a GLP-1 dose that always falls within ±10% of a given "fixed" dose.

Table 7 (shown below), for example, shows a family or system of 6 min/max pen-type injection devices that allow the delivery of any long acting insulin dose from 22-76 units along with a GLP-1 dose that is "fixed" to 20mg ±10%. Each Pen within the family would have different minimum and maximum dose thresholds and would be provided with a primary pack or cartridge of medicament filled with the appropriate mix ratio of the two medicines. The family of pen devices could be provided as disposable mechanical devices, prefilled with the appropriate mix ratio cartridge of medicament. Alternatively, the family of devices could be provided as reusable mechanical devices. In the latter case, the devices would be preferably dedicated to a particular mixed ratio cartridge, i.e. only the correct mix ratio cartridge can be loaded into each pen family member.

A third alternative is to provide the family of pen devices via a single electronic device that can be programmed with the minimum and maximum dose functionality. Preferably, the min/max electronic device would be loaded with a coded cartridge that would automatically upon being loaded into the device communicate to the device what the required minimum and maximum thresholds should be for that particular cartridge and mix ratio.

There are many of ways of achieving a minimum and/or maximum settable dose on a rotationally driven, variable dose, drug delivery device, such as a pen-type device. For example, dose setting modules on such injection devices could contain the following mechanisms:
1. a limiting dose dial sleeve that can fit over a dose dial sleeve to limit the maximum dose that can be dialed. The axial length of the limiting sleeve can be varied to set the predetermined maximum allowable dose.
2. a clutch nut mechanism that prevents dosing of the device until a predetermined minimum dose has been set. A clutch blocking mechanism that prevents the clutch from releasing the dose dial sleeve during injection until a minimum dose has been set.
3. a combined helical and axial clutch path whereby setting a predetermined minimum dose causes the clutch to travel in a predetermined axial and helical path to unlock the device to allow delivery.
4. one or more flexible components in the dose setting module that changes its geometry after a predetermined minimum dose has be dialed or set, thus unlocking or freeing up the dose setting module to allow a user to deliver the set dose. The minimum predetermined dose can be adjusted by altering the travel distance of the flexible components.

These as well as other advantages of various aspects of the proposed drug delivery device will become apparent to those of ordinary skill in the art by reading the following detailed description, with appropriate reference to the accompanying drawings.

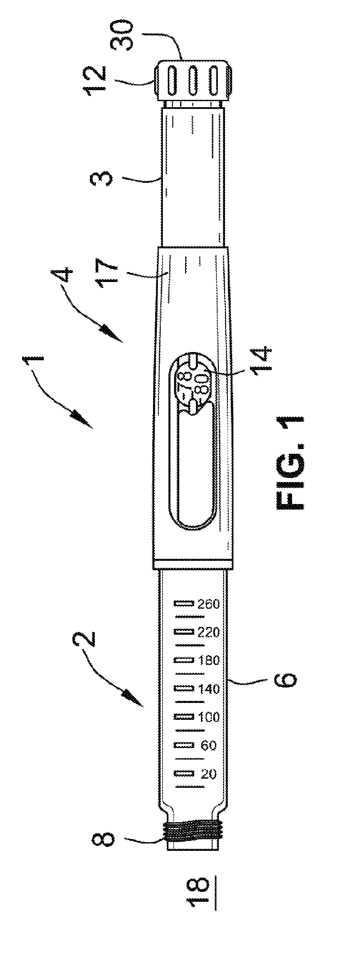

Figure 1 illustrates a generic design of a pen-type drug delivery device capable of accepting the min/max functionality of the present invention.

Referring to Figure 1, there is shown a drug delivery device 1 in accordance with an exemplary pen-type design arrangement. The drug delivery device 1 comprises a housing having a first cartridge retaining part 2, and a dose setting mechanism 4 which might be provided in a proximal housing part 17. The drug delivery device may be a resettable drug delivery device (i.e., a reusable device) or alternatively a non-resettable drug delivery device (i.e., a non-reusable device). A first end of the cartridge retaining part 2 and a second end of proximal housing part 17 or the dose setting mechanism 4 are secured together by connecting features. For non-resettable devices, these connecting features would be permanent and non-reversible. For resettable devices, these connecting features would be releasable.

In this illustrated arrangement, the cartridge housing 2 is secured within the second end of the dose setting mechanism 4. A removable cap (not shown) is releasably retained over a second end or distal end of a cartridge retaining part or cartridge housing. The dose setting mechanism 4 comprises a dose setting element 3 with a dose dial grip 12 arranged on a proximal end 30 of the device and a window or lens 14. A dose scale arrangement is viewable through the window or lens 14. To set a dose of medication contained within the drug delivery device 1, a user rotates the dose dial grip 12 such that a dialed dose will become viewable in the window or lens 14 by way of the dose scale arrangement.

Figure 1 illustrates the medical delivery device 1 with the cover cap removed from a distal end 18 of the medical delivery device 1. This removal exposes the cartridge housing 6. Preferably, a cartridge (not shown) from which a number of doses of a medicinal product may be dispensed, is provided in the cartridge housing 6. Preferably, the cartridge contains a type of medicament that can be administered relatively often, such as once or more times a day. One such medicament is either long acting or short acting insulin or an insulin analog. The cartridge comprises a bung or stopper that is retained near a second end or a proximal end of the cartridge. The medical delivery device also comprises a driver having a spindle (not illustrated in Figure 1). As discussed above, before the device is primed, there may or may not be a gap between the end of the spindle and the cartridge bung (not shown in Figure 1).

The cartridge housing 6 has a distal end and a proximal end. Preferably, the distal end of the cartridge housing 6 comprises a hub 8 for attaching a removable needle assembly. However, other needle assembly connection mechanisms could also be used. If the drug delivery device 1 comprises a resettable device, the cartridge proximal end is removably connected to the dose setting mechanism 4. In one preferred embodiment, cartridge housing proximal end is removably connected to the dose setting mechanism 4 via a bayonet connection. However, as those of ordinary skill in the art will recognize, other types of removable connection methods such as threads, partial threads, ramps and detents, snap locks, snap fits, and luer locks may also be used.

As previously mentioned, the dose setting mechanism 4 of the drug delivery device illustrated in Figure 1 may be utilized as a reusable drug delivery device. (i.e., a drug delivery device that can be reset) Where the drug delivery device 1 comprises a reusable drug delivery device, the cartridge is removable from the cartridge housing 6. The cartridge may be removed from the device 1 without destroying the device 1 by merely having the user disconnect the dose setting mechanism 4 from the cartridge housing 6.

In use, once the cap is removed, a user can attach a suitable needle assembly to the hub 8 provided at the distal end of the cartridge housing 6. Such needle assembly may be, for example, screwed onto a distal end of the housing 6 or alternatively may be snapped onto this distal end. After use, the replaceable cap may be used to re-cover the cartridge housing 6. Preferably, the outer dimensions of the replaceable cap are similar or identical to the outer dimensions of the dose setting mechanism 4 so as to provide an impression of a unitary whole when the replaceable cap is in position covering the cartridge housing 6 when the device is not in use.

Using pen-type devices of the design illustrated in Figure 1, the invention embodies a drug delivery device comprising a medicament reservoir attached to a dose setting module, where the dose setting module is designed to allow a user to set a dose of medicament from zero to a maximum achievable designed dose, however, the dose setting module is specifically configured to only allow delivery of a set dose if the set dose is equal to or greater than a predetermined minimum dose X of the medicament and where the dose setting module is configured to prevent a user from setting a dose that exceeds a predetermined maximum dose Y of the medicament, where X is greater than zero and Y is less than a maximum achievable designed dose setting of the injection device. For example, in the case of a pen device for insulin delivery, that maximum achievable designed dose is usually 60 or 80 units. Further, it is preferred that the achievable dosage range, from dose Y to dose X, equals a therapeutic range of medicament prescribed by a physician.

The invention also relates to a system or kit of two or more such drug delivery devices where a first drug delivery device having a dose setting module is configured to allow dose delivery only when a set dose exceeds a predetermined minimum dose X and is less than a predetermined maximum dose Y. The system also contains at least a second drug delivery device having a dose setting module configured to allow dose delivery only when a set dose exceeds a predetermined minimum dose of greater than X, but less than Y, and the dose does not exceed a predetermined maximum dose that is greater than Y. Preferably, the system could have three or more delivery devices where the third device has a dose setting module configured to allow dose delivery only when a set dose exceeds a predetermined minimum dose that is greater than the minimum dose of the second drug delivery device and less than the maximum dose of the second drug delivery device. More preferably the dose setting module of the third drug delivery device could have a predetermined maximum dose greater than the maximum dose of the second drug delivery device. So for example, referring to Table 1, the first pen in the system could have X equal 10 units and Y could equal 24. The second pen in the system could have a set minimum dose of 22 and a maximum dose of 40. The third pen would have a predetermined minimum dose of 36 and a maximum of 60, and so on.

In another preferred embodiment the difference between the minimum dose and the maximum dose of each drug delivery device is predetermined to substantially equal to a prescribed therapy amount of medicament for a patient using the drug delivery devices. It is also preferable that each pen in the system is configured to allow a predetermined priming dose of medicament. In yet another embodiment of the invention each drug delivery device contains a reservoir of medicament comprising a least two active ingredient that are identical, and most preferably each reservoir contains a different ratio of the two active ingredients.

In a preferred embodiment a master drug compound, such as insulin, contained within a multiple dose, user selectable device could be used with a single use, user replaceable, module that contains a single dose of a secondary medicament and the single dispense interface. When connected to the primary device, the secondary compound is activated/delivered on dispense of the primary compound. Although the present application specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with the present invention.

For the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta-decanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH2).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

Exemplary embodiments of the present drug delivery device have been described. Those skilled in the art will understand, however, that changes and modifications may be made to these embodiments without departing from the true scope and spirit of the presently proposed drug delivery device, which is defined by the claims.

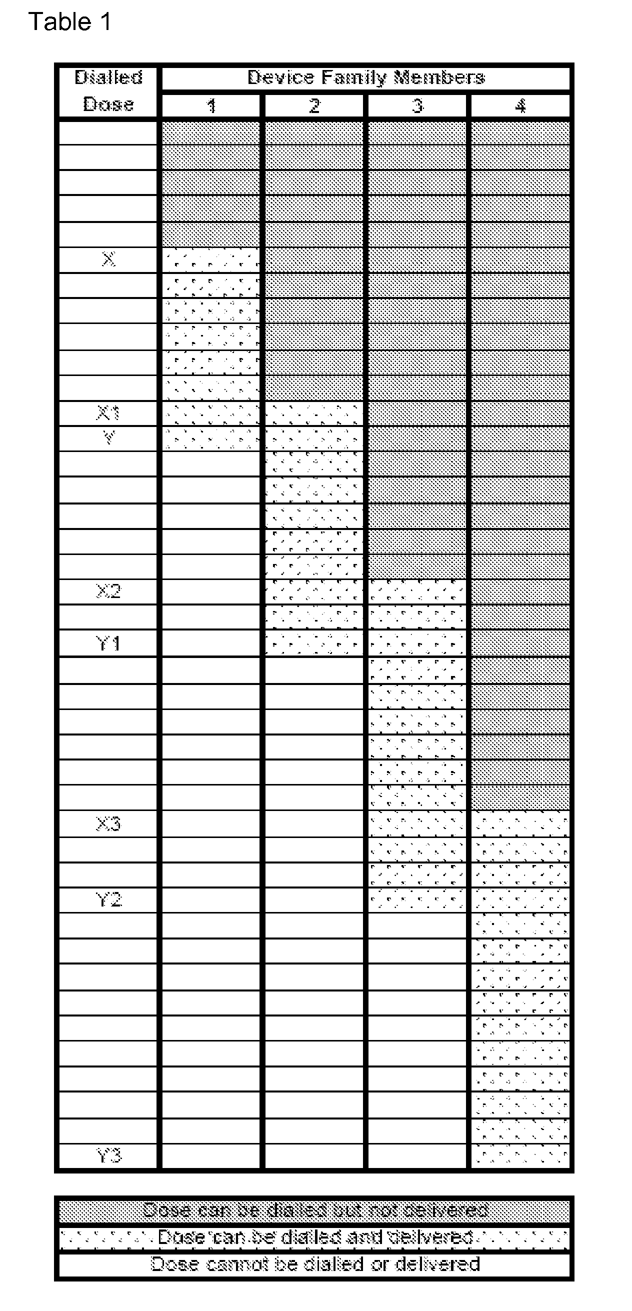

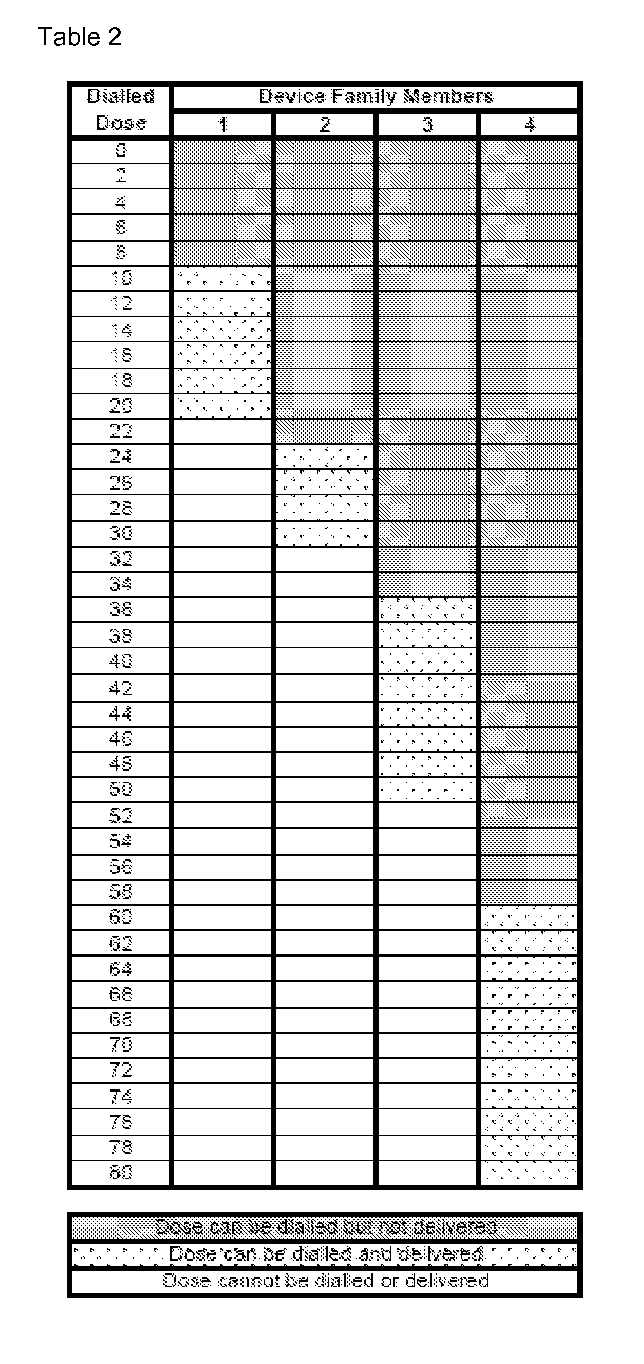

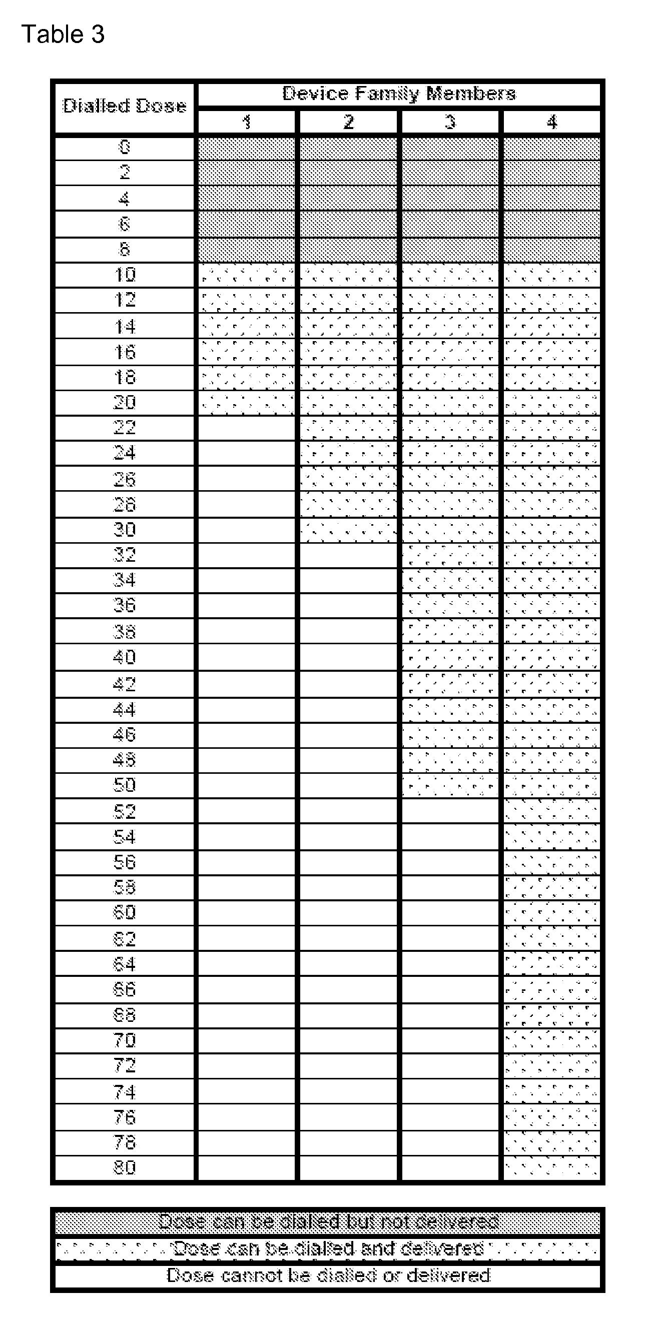

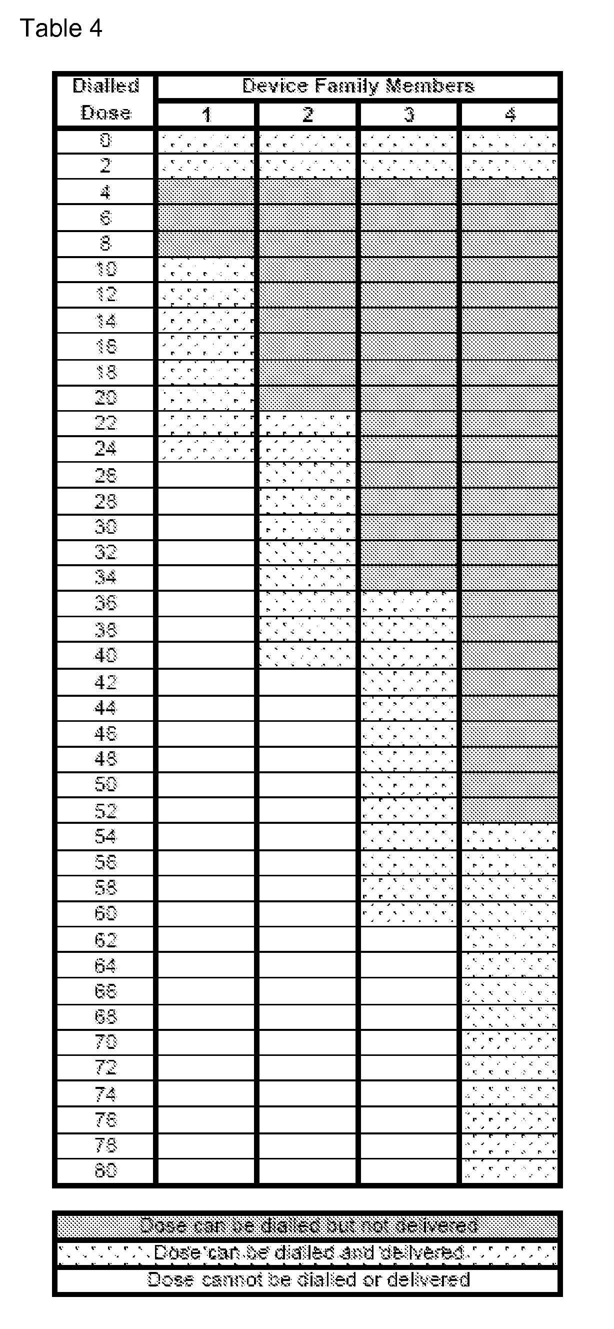

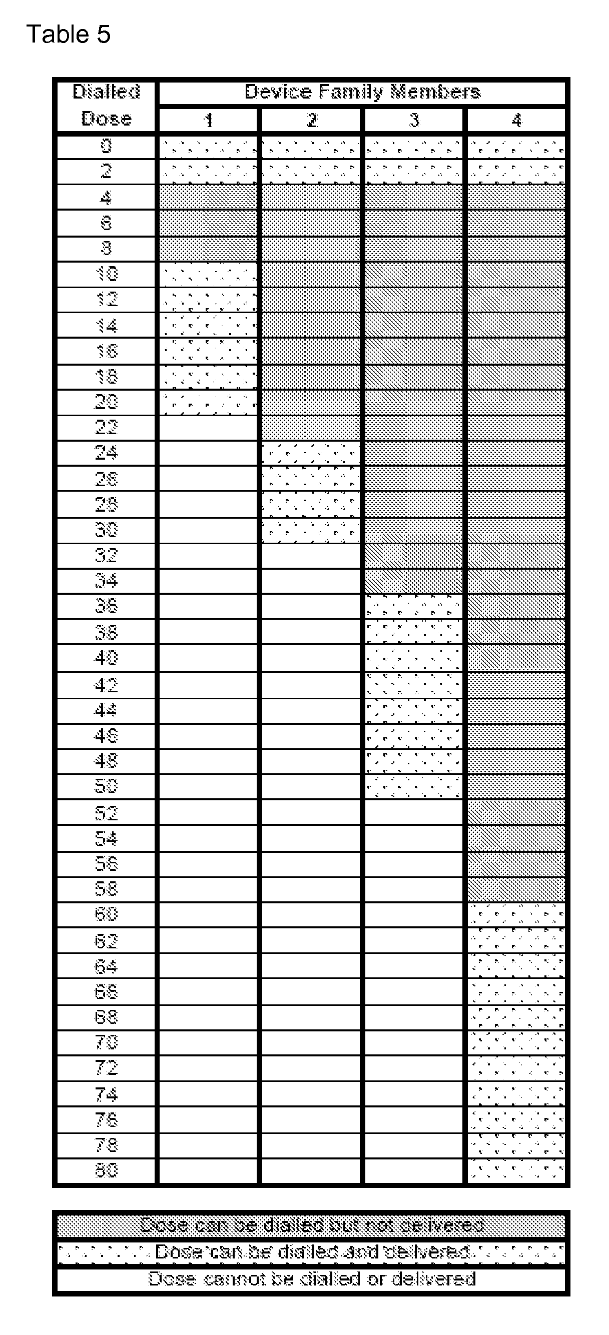

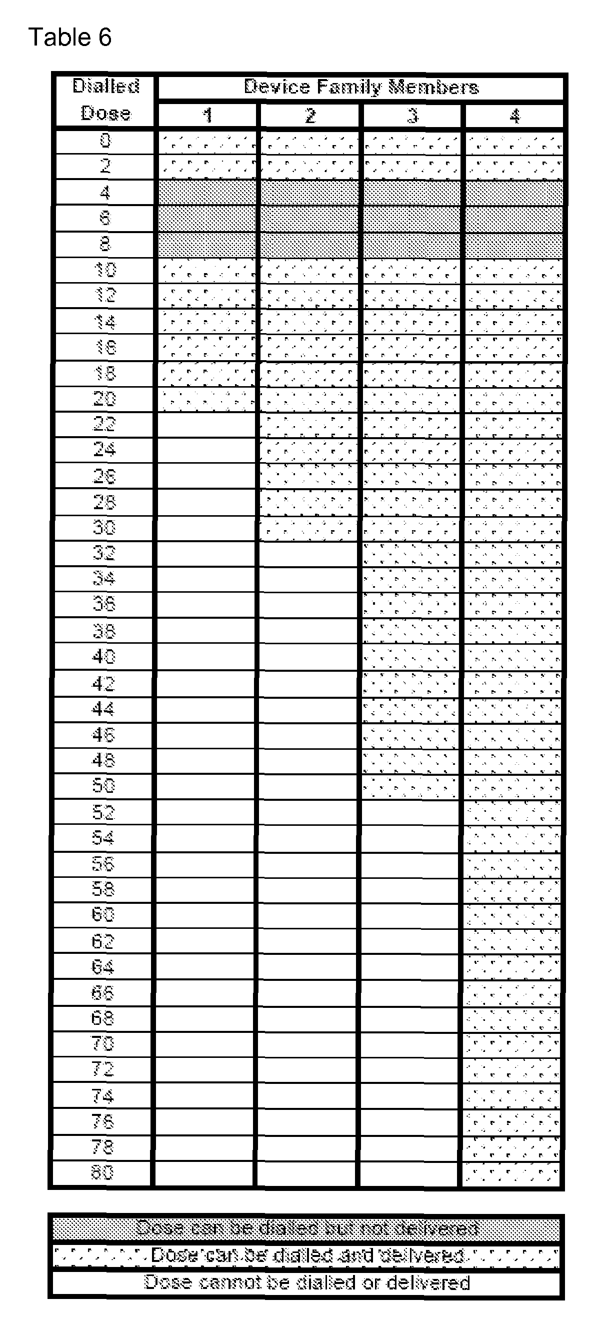

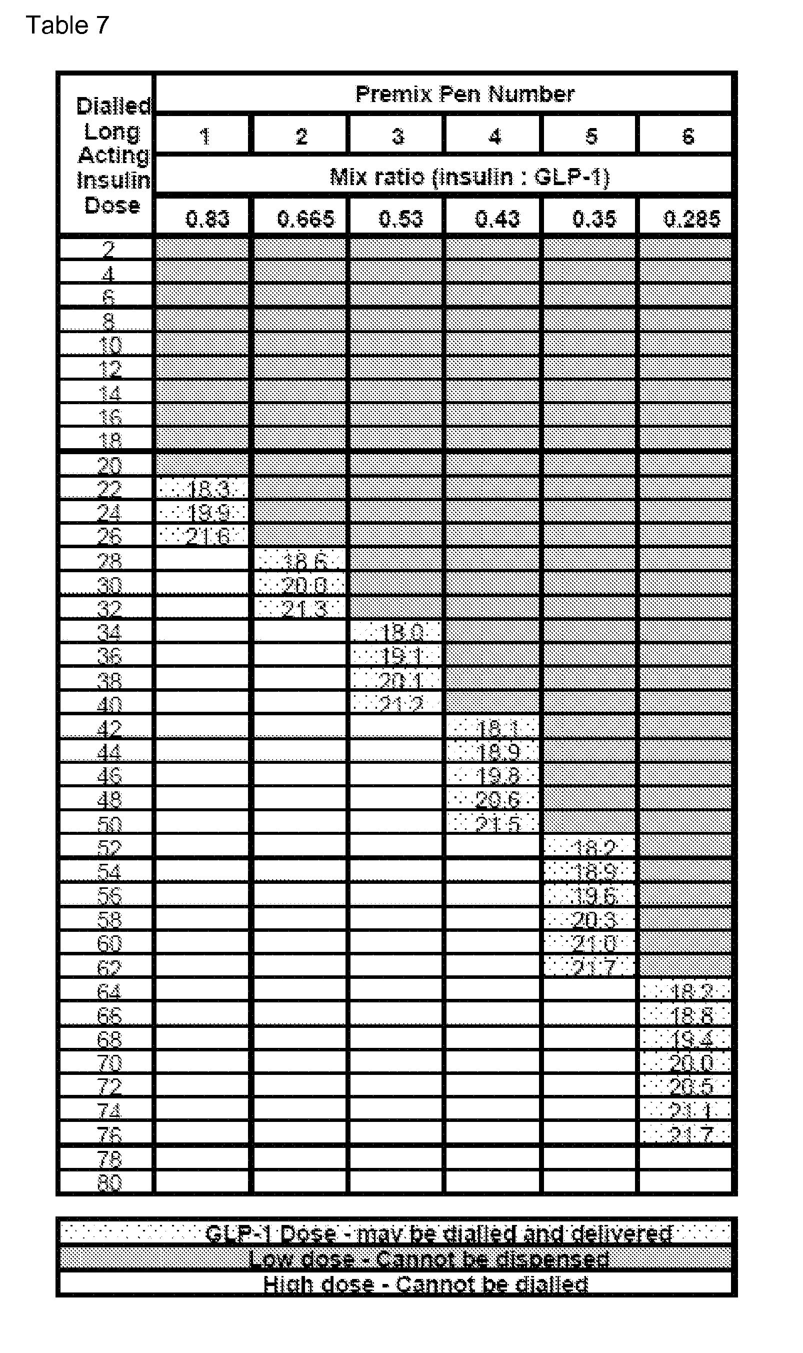

## Claims

1. Drug delivery device comprising:
a medicament reservoir (6) attached to a dose setting module (4), where the dose setting module (4) is configured to only allow delivery of a set dose if the set dose is equal to or greater than a predetermined minimum dose X of the medicament and
where the dose setting module (4) is configured to prevent a user from setting a dose that exceeds a predetermined maximum dose Y of the medicament, where X is greater than zero.

2. Drug delivery device of claim 1 where dose Y must be less than a maximum achievable designed dose setting of the delivery device (1).

3. Drug delivery device according to claims 1 or 2, wherein the dose setting module (4) comprises a dose dial sleeve and a limiting dose dial sleeve that can fit over said dose dial sleeve to limit the maximum dose that can be dialed.

4. Drug delivery device according to claims 1 or 2, wherein the dose setting module (4) comprises a clutch nut mechanism that prevents dosing of the device until a predetermined minimum dose has been set.

5. Drug delivery device according to claims 1 or 2, wherein the dose setting module (4) comprises a combined helical and axial clutch path whereby setting a predetermined minimum dose causes the clutch to travel in a predetermined axial and helical path to unlock the device to allow delivery of the medicament.

6. Drug delivery device according to claims 1 or 2, wherein the dose setting module (4) comprises at least one flexible component that changes its geometry after a predetermined minimum dose has be dialed or set, thus unlocking or freeing up the dose setting module (4) to allow a user to deliver the set dose.

7. System of two or more drug delivery devices according to any of the preceding claims wherein a first drug delivery device (1) and a second drug delivery device (1) each have a different therapeutic range of dose X to dose Y.

8. System of claim 7 wherein the therapeutic range of the first drug delivery device (1) and the therapeutic range of the second drug delivery device (1) partially overlap.

9. System of claim 7 wherein the therapeutic range of the first drug delivery device (1) and the therapeutic range of the second drug delivery device (1) do not overlap.

10. System of claim 7 wherein the therapeutic range of the first drug delivery device (1) is a subset of the therapeutic range of the second drug delivery device (1).

11. System according to any of claims 7 to 10wherein each drug delivery device (1) has a minimum dose equal to X.

12. System according to any of claims 7 to 11 where the dose setting module (4) of each drug delivery device (1) is configured to allow a predetermined priming dose of medicament.

13. System according to any of claims 7 to 12where each reservoir (6) contains medicament comprising two active ingredients.

14. System of claim 13 where each reservoir (6) contains a different ratio of the two active ingredients.

15. System according to any of claims 7 to 14where each drug delivery device (1) contains a reservoir (6) of medicament comprising insulin and GLP-1.
